(19) Europäisches Patentamt
European Patent Office
Office européen des brevets

(11) EP 1 391 448 A1

(12) EUROPÄISCHE PATENTANMELDUNG

(43) Veröffentlichungstag:
**25.02.2004 Patentblatt 2004/09**

(21) Anmeldenummer: **03018220.8**

(22) Anmeldetag: **11.08.2003**

(51) Int Cl.[7]: **C07C 29/143**, C07C 67/56, C07C 237/06, C07C 255/25, C07F 9/40, C07F 15/00

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **21.08.2002 DE 10238114**

(71) Anmelder: **Bayer Chemicals AG**
**51368 Leverkusen (DE)**

(72) Erfinder: **Köcher, Jürgen, Dr.**
**40764 Langenfeld (DE)**

(54) **Optisch aktive Diamine und deren Anwendung in katalytischen Prozessen**

(57) Die Erfindung betrifft stereoisomerenangereicherte Diamine, der Formel (I),

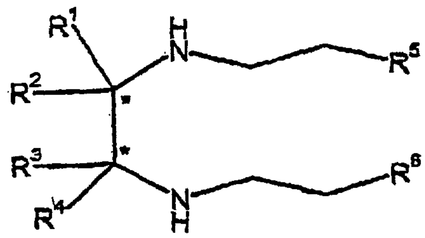

(I),

in der

| | |
|---|---|
| * | stereogene Kohlenstoffatome markiert, die jeweils unabhängig voneinander R-oder S-konfiguriert sind, wobei jedoch Meso-Formen ausgeschlossen sind und |
| $R^1$,$R^2$,$R^3$ und $R^4$ | jeweils unabhängig voneinander für Wasserstoff, $C_1$-$C_{12}$-Alkyl, $C_4$-$C_{24}$-Aryl oder $C_5$-$C_{25}$-Arylalkyl stehen oder $R^1$,$R^2$,$R^3$ und $R^4$ zusammen mit der Ethylenbrücke für 1,2-($C_5$-$C_8$-Cycloalkyl) stehen und |
| $R^5$ und $R^6$ | jeweils unabhängig voneinander für Reste stehen, die ausgewählt sind aus der Gruppe -$COOR^7$, -$CONR^8R^9$, -CN oder -$PO(OR^{10})_2$, wobei $R^7$,$R^8$,$R^9$ und $R^{10}$ jeweils für $C_1$-$C_{12}$-Alkyl, $C_4$-$C_{24}$-Aryl oder $C_5$-$C_{25}$-Arylalkyl stehen oder $NR^8R^9$ als Ganzes für einen cyclischen Aminorest mit insgesamt 4 bis 12 Kohlenstoffatomen steht |

Metallkomplexe, enthaltend diese Diamine sowie deren Anwendung in einem Verfahren zur asymmetrischen Reduktion von Ketonen unter Anwendung von Silanen wie insbesondere Polymethylhydrosiloxan als Reduktionsmittel.

EP 1 391 448 A1

## Beschreibung

Die Erfindung betrifft stereoisomerenangereicherte Diamine, Metallkomplexe, enthaltend diese Diamine sowie deren Anwendung in einem Verfahren zur asymmetrischen Reduktion von Ketonen unter Anwendung von Silanen wie insbesondere Polymethylhydrosiloxan als Reduktionsmittel.

Aufgrund der stetig wachsenden Bedeutung von stereoisomerenangereicherten Alkoholen als Feinchemikalien, Wirkstoffe in Agrochemikalien und Pharmazeutika oder deren Zwischenprodukte sowie Aromen und Riechstoffe hat die asymmetrische Reduktion von Ketonen technische Anwendung im industriellen Maßstab gefunden.

Dabei ist der Focus insbesondere auf die Entwicklung von Katalysatoren gerichtet, die bei hohen Stereoselektivitäten hohe Ausbeuten und Umsatzraten ermöglichen.

Aus US-Patent 5,227,538 und J. Am. Chem. Soc. 116 (1994), 11667 sind Verfahren bekannt, bei denen die asymmetrische Reduktion von Ketonen mit Silanen in Gegenwart eines Titan-Komplexes mit optisch aktiven Liganden als Katalysator durchgeführt wird. Nachteil dieser Verfahren ist der hohe Preis der Katalysatoren bei hohem Bedarf, der im Bereich von 5 Mol- % bezogen auf das eingesetzte Keton beträgt.

Aus WO-A 99/50211, WO-A 99/12877 und J. Am. Chem. Soc. 1999, 121, 6158 sind Verfahren zur asymmetrischen Reduktion von Ketonen bekannt, in denen als preiswertes Reduktionsmittel Polymethylhydrosiloxan (PHMS) und als Katalysatoren Metallhydride eingesetzt werden, die aus einem Metallsalz oder einem Metallkomplex durch ein Reduktionsmittel gebildet werden. Die asymmetrische Induktion erfolgt dabei durch stereoisomerenangereicherte, sekundäre 1,2-Diamine als Liganden. Ein Nachteil dieser Methode ist die Tatsache, dass die mit diesen Verfahren erzielten optischen Reinheiten der Alkohole mit 70 bis 80 % nur moderat sind.

Es bestand daher das Bedürfnis, weitere Katalysatoren und Liganden zu entwickeln, die die asymmetrische Reduktion von Ketonen mit guten Ausbeuten und guten optischen Reinheiten ermöglichen.

Es wurden nun Verbindungen der Formel (I) gefunden

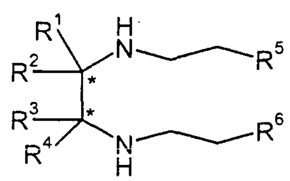

I,

in der

| | |
|---|---|
| * | stereogene Kohlenstoffatome markiert, die jeweils unabhängig voneinander R- oder S-konfiguriert sind, wobei jedoch Meso-Verbindungen ausgeschlossen sind, |
| $R^1$, $R^2$, $R^3$ und $R^4$ | jeweils unabhängig voneinander für Wasserstoff, $C_1$-$C_{12}$-Alkyl, $C_4$-$C_{24}$-Aryl oder $C_5$-$C_{25}$-Arylalkyl stehen oder $R^1$, $R^2$, $R^3$ und $R^4$ zusammen mit der Ethylenbrücke für 1,2-($C_5$-$C_8$-Cycloalkyl) stehen und |
| $R^5$ und $R^6$ | jeweils unabhängig voneinander für Reste stehen, die ausgewählt sind aus der Gruppe $-COOR^7$, $-CONR^8R^9$, -CN oder $-PO(OR^{10})_2$, wobei $R^7$, $R^8$, $R^9$ und $R^{10}$ jeweils für $C_1$-$C_{12}$-Alkyl, $C_4$-$C_{24}$-Aryl oder $C_5$-$C_{25}$-Arylalkyl stehen oder $NR^8R^9$ als Ganzes für einen cyclischen Aminorest mit insgesamt 4 bis 12 Kohlenstoffatomen steht. |

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen, in beliebiger Weise kombiniert werden.

Die Verbindungen der Formel (I) besitzen verschiedene Stereoisomere. Von der Erfindung sind sowohl die reinen Stereoisomere als auch beliebige Mischungen von Stereoisomeren wie zum Beispiel Racemate umfasst.

**Alkyl** bedeutet jeweils unabhängig einen geradkettigen, cyclischen, verzweigten oder unverzweigten Alkyl-Rest. Gleiches gilt für den nichtaromatischen Teil eines Arylalkyl-Restes.

$C_1$-$C_4$-Alkyl steht beispielsweise für Methyl, Ethyl, n-Propyl, iso-Propyl, n-Butyl, sec.-Butyl und tert.-Butyl, $C_1$-$C_8$-Alkyl darüber hinaus beispielsweise für n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1 -Ethyl-2-methylpropyl, 1-Ethyl-2-methylpropyl, n-Heptyl und n-Octyl, $C_1$-$C_{12}$-Alkyl weiter darüber hinaus beispielsweise für Adamantyl, n-Nonyl, n-Decyl und n-Dodecyl.

**Aryl** bedeutet jeweils unabhängig einen heteroaromatischen Rest mit 4 bis 24 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 24 Gerüstkohlenstoffatomen.

Beispiele für carbocyclische aromatische Reste mit 6 bis 24 Gerüstkohlenstoffatomen sind Phenyl, Naphtyl, Phenanthrenyl, Anthracenyl oder Fluorenyl, heteroaromatische Reste mit 4 bis 24 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, sind beispielsweise Pyridinyl, Benzofüranyl, Dibenzofuran-yl oder Chinolinyl.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Chlor, Fluor, $C_1$-$C_{12}$-Alkyl, $C_1$-$C_{12}$-Alkoxy, Di($C_1$-$C_8$-alkyl)amino, COO($C_1$-$C_8$-Alkyl), CON($C_1$-$C_8$-Alkyl)$_2$, COO($C_1$-$C_8$-Alkyl), COO ($C_4$-$C_{14}$-Aryl), CO($C_1$-$C_8$-Alkyl), $C_5$-$C_{15}$-Arylalkyl oder Tri($C_1$-$C_6$-alkyl)-siloxyl.

$C_4$-$C_{24}$-Aryl steht beispielsweise und bevorzugt für Phenyl, o-, p-, m-Tolyl, o-, p-, m-Anisyl, o-, p-, m-Fluorphenyl, o-, p-, m-Chlorphenyl, o-, p-, m-Trifluormethylphenyl, o-, p-, m-Nitrophenyl und 2-, 3- und 4-Pyridyl.

**Arylalkyl** bedeutet jeweils unabhängig einen geradkettigen, cyclischen, verzweigten oder unverzweigten Alkyl-Rest nach obiger Definition, der einfach, mehrfach oder vollständig durch Aryl-Reste gemäß obiger Definition substituiert sein kann.

$C_5$-$C_{15}$-Arylalkyl steht beispielsweise und bevorzugt für Benzyl oder (R)- oder (S)-1-Phenylethyl.

Im Folgenden werden die bevorzugten Substitutionsmuster für Verbindungen der Formel (I) definiert:

$R^1$, $R^2$, $R^3$ und $R^4$ stehen bevorzugt jeweils unabhängig voneinander für Wasserstoff, $C_1$-$C_8$-Alkyl oder $C_4$-$C_{24}$-Aryl oder $R^1$, $R^2$, $R^3$ und $R^4$ zusammen mit der Ethylenbrücke für 1,2-Cyclohexylen.

$R^1$, $R^2$, $R^3$ und $R^4$ stehen besonders bevorzugt zusammen mit der Ethylenbrücke für 1,2-Dimethyl-1,2-ethylen, 1,2-Diphenyl-1,2-ethylen oder 1,2-Cyclohexylen, ganz besonders bevorzugt für (R,R)- und (S,S)-1,2-Diphenyl-1,2-ethylen oder (R,R)- und (S,S)-1,2-Cyclohexylen.

$R^5$ und $R^6$ stehen bevorzugt jeweils unabhängig voneinander, besonders bevorzugt jeweils identisch für Reste, die ausgewählt sind aus der Gruppe -COOR$^7$, -CONR$^8$R$^9$, -CN oder -PO(OR$^{10}$)$_2$, wobei $R^7$, $R^8$, $R^9$ und $R^{10}$ jeweils für $C_1$-$C_4$-Alkyl oder $C_4$-$C_{24}$-Aryl stehen.

$R^5$ und $R^6$ stehen ganz besonders bevorzugt für $-COOCH_3$, $-COOC_2H_5$, -CN, $-CON(CH_3)_2$, $-CON(C_2H_5)_2$, -PO $(OCH_3)_2$, $-PO(OC_2H_5)_2$ und -PO(OPhenyl)$_2$.

Als ganz besonders bevorzugte Verbindungen der Formel (I) seien genannt:

(1S,2S)- und (1R,2R)-Bis-[N-(2-dimethylphosphonato-ethyl)amino]-cyclohexan,
(1S,2S)- und (1R,2R)-Bis-[N-(2-diethylphosphonato-ethyl)amino]-cyclohexan,
(1S,2S)- und (1R,2R)-Bis-[N-(2-diphenylphosphonato-ethyl)amino]-cyclohexan,
(1S,2S)- und (1R,2R)-Bis-[N-(2-cyanoethyl)amino]-cyclohexan, (1S,2S)- und
(1R,2R)-Bis-[N-(2-carboxylethyl-ethyl)amino]-cyclohexan, und (1S,2S)- und
(1R,2R)-Bis-[N-(2-carboxylmethyl-ethyl)amino]-cyclohexan, (1S,2S)- und (1R,2R)-Bis-[N-(2-dimethylphosphonato-ethyl)amino]-1,2-diphenylethan, (1S,2S)- und
(1R,2R)-Bis-[N-(2-diethylphosphonato-ethyl)amino]-1,2-diphenylethan, (1S,2S)-und (1R,2R)-Bis-[N-(2-diphenylphosphonato-ethyl)amino]-1,2-diphenylethan,
(1S,2S)- und (1R,2R)-Bis-[N-(2-cyanoethyl)amino]-1,2-diphenylethan, (1S,2S)- und
(1R,2R)-Bis-[N-(2-carboxyethyl-ethyl)amino]-1,2-diphenylethan, (1S,2S)- und (1R, 2R)-Bis-[N-(2-carboxymethyl-ethyl)amino)-1,2-diphenylethan.

Die erfindungsgemäßen Verbindungen der Formel(I) können in an sich bekannter Weise (siehe WO-A 00/24751) beispielsweise durch Umsetzung von Aminen der Formel (II)

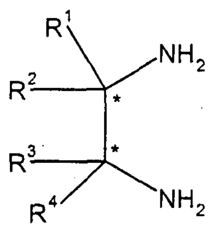

(II),

in der

*, $R^1$, $R^2$, $R^3$ und $R^4$ die unter der Formel (I) genannten Bedeutungen einschließlich deren Vorzugsbereichen besitzen, mit Verbindungen der Formel (III)

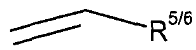

(III),

in der

$R^5$ und $R^6$ die unter der Formel (I) genannten Bedeutungen einschließlich deren Vorzugsbereichen besitzen, hergestellt werden.

Von der Erfindung sind weiterhin Katalysatoren umfasst, die Übergangsmetallkomplexe von Verbindungen der Formel (I) enthalten.

Vorzugsweise beträgt das Verhältnis von Übergangsmetall zu Verbindung der Formel (I) dabei 0,05 bis 2,0, besonders bevorzugt 0,1 bis 1,5 und ganz besonders bevorzugt 0,9 bis 1,1.

Übergangsmetallkomplexe von Verbindungen der Formel (I) sind bevorzugt Zinkund Cobalt-Komplexe von Verbindungen der Formel (I).

Besonders bevorzugte Übergangsmetallkomplexe von Verbindungen der Formel (I) sind solche, die durch Umsetzung von Zink- oder Kobalt-Halogeniden, Carbonaten, Cyanuraten, Isocyanaten, Sulfaten, Phosphaten, Nitraten, Carboxylaten oder Alkoxiden mit Verbindungen der Formel (I) erhältlich sind.

Solche Übergangsmetallkomplexe werden bevorzugt vor dem Einsatz als Katalysator mit einem Reduktionsmittel umgesetzt. Geeignete Reduktionsmittel sind beispielsweise Hydride von Alkali- und Erdalkalimetallen oder Aluminium-Wasserstoff- oder Bor-Wasserstoff-Verbindungen wie insbesondere solche der Formel (IV)

$$Met[EH_qR^{11}_{(4-q)}]_p \qquad (IV),$$

in der

Met für ein ein- oder zweiwertiges Metall wie vorzugsweise Zink, Lithium, Natrium oder Kalium und

E für Aluminium oder Bor und

$R^{11}$ für $C_1$-$C_8$-Alkyl und

q für 1, 2, 3 oder 4, bevorzugt für 4 oder 1, und

p für die Wertigkeit von Met stehen.

Ganz besonders bevorzugte Reduktionsmittel sind $LiBH_4$, $NaBH_4$, $Zn(BH_4)_2$, $LiAlH_4$, Li[BHEthyl$_3$] und Li[AlH (sek.-butyl)3], Na[AlH$_2$(2-methoxyethoxy)2], Natriumhydrid und Lithiumhydrid.

Die Menge des Reduktionsmittels wird vorzugsweise so gewählt, dass das molare Verhältnis von hydridischem Wasserstoff in den Reduktionsmitteln und dem Übergangsmetallkomplex 0,5 bis 5 beträgt. Größere Verhältnisse sind möglich, aber unwirtschaftlich.

Weitere besonders bevorzugte Übergangsmetallkomplexe sind solche, die durch Umsetzung von Verbindungen der Formel (I) mit Zinkverbindungen $ZnY_2$ oder ZnYHal erhalten werden, wobei Y jeweils unabhängig für Wasserstoff, $BH_4$ oder einen organischen Rest wie beispielsweise und bevorzugt $C_1$-$C_8$-Alkyl oder Phenyl steht und Hal für Brom, Chlor oder Iod steht.

Bevorzugte Zinkverbindungen $ZnY_2$ sind Dimethylzink, Diethylzink, Dibutylzink, Diphenylzink und Zinkhydrid. Bevorzugte Zinkverbindungen ZnYHal beispielsweise Phenylzinkchlorid, Methylzinkchlorid und Ethylzinkchlorid.

Diese weiteren bevorzugten Übergangsmetallkomplexe können direkt als Katalysatoren verwendet werden.

Von der Erfindung ist weiterhin ein Verfahren zur asymmetrischen Reduktion von Ketonen mit Silanen in Gegenwart von Katalysatoren umfasst, das dadurch gekennzeichnet ist, dass als Katalysatoren solche eingesetzt werden, die Übergangsmetallkomplexe von Verbindungen der Formel (I) enthalten.

Die für die Übergangsmetallkomplexe und für die Verbindungen der Formel (I) genannten Bedeutungen, einschließlich deren Vorzugsbereiche, gelten dabei entsprechend.

Übergangsmetallkomplexe von Verbindungen der Formel (I) können entweder als isolierte Komplexe eingesetzt werden oder in einer bevorzugten Verfahrensweise direkt im Reaktionsgemisch hergestellt werden.

Als Silane sind bevorzugt solche der Formel (V)

$$H_rSiCl_s(C_1\text{-}C_8\text{-Alkyl})t(C_1\text{-}C_8\text{-Alkoxy})_u(\text{Phenyl})_v \qquad (V),$$

in der

r eins, zwei oder drei ist

und

$$(s + t + u + v) = (4\text{-}r)$$

ist

oder Polymethylhydrosiloxan (PMHS) mit der wiederkehrenden Struktureinheit

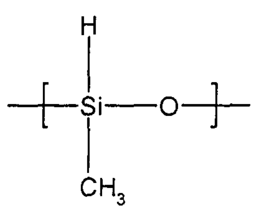

Besonders bevorzugt sind $H_2Si(C_1\text{-}C_8\text{-Alkyl})_2$, $HSi(C_1\text{-}C_8\text{-Alkoxy})_3$, $H_rSiCl_s(C_1\text{-}C_8\text{-Alkyl})_t$ $HSiPhenyl_3$, $H_2SiPhenyl_2$, $H_3SiPhenyl$ und PMHS, wobei PMHS noch weiter bevorzugt ist.

Die Menge des Katalysators wird vorteilhafterweise so gewählt, dass das molare Verhältnis von Übergangsmetall, bevorzugt Zink, zu eingesetztem Keton 0,001 bis 0,20, bevorzugt 0,01 bis 0,1 und besonders bevorzugt 0,01 bis 0,05 beträgt.

Die Reaktionstemperatur beträgt beispielsweise -20°C bis 120°C, vorzugsweise 0°C bis 60°C.

Die Reaktion kann mit oder ohne, vorzugsweise mit Lösungsmittel durchgeführt werden. Als Lösungsmittel können zum Beispiel Ether wie Methyl-tert.-butylether, Dioxan, Tetrahydrofüran, Ethylenglykoldimethylether oder Diisopropylether sowie aliphatische oder aromatische Kohlenwasserstoffe wie beispielsweise Cyclohexan, Toluol, n-Hexan, n-Heptan, Petrolether, Xylol oder Mesitylen verwendet werden.

Bevorzugte Ketone sind Arylketone wie insbesondere gegebenenfalls substituierte Acetophenone, Propiophenone oder Butyrophenone.

Auf erfindungsgemäße Weise werden stereoisomerenangereicherte Alkohole erhalten.

Die erfindungsgemäß herstellbaren stereoisomerenangereicherten Alkohole eignen sich insbesondere in einem Verfahren zur Herstellung von Arzneimitteln und Agrochemikalien.

Der Vorteil der Erfindung liegt darin, dass Liganden und deren Übergangsmetallkomplexe zur Verfügung gestellt werden, die in einfacher Weise herstellbar sind und in der asymmetrischen Reduktion von Ketonen hohe Ausbeuten und Stereoselektivitäten erlauben.

**Beispiele**

**Beispiel 1:**

**Herstellung von (1S,2S)-Bis-[N-(2-cyanoethyl)-amino]-cyclohexan**

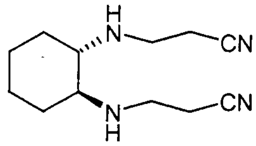

1,0 g (1S,2S)-(+)-Diaminocyclohexan (8.8 mmol) werden bei Raumtemperatur in 10 ml Ethanol gelöst. Man fügt 1,24 ml Acrylnitril (1,0 g, 18,8 mmol) hinzu und rührt 72 h bei Raumtemperatur. Laut gaschromatographischer Analyse ist das Diaminocyclohexan vollständig umgesetzt worden. Nach Destillation des Alkohols verbleibt ein farbloses Öl (1,8 g), welches laut gaschromatographischer Analyse ein einheitliches Produkt darstellt (Reinheit > 98 %).

**Beispiel 2:**

**Herstellung von (1S,2S)-Bis- [N-(2-cyanoethyl)-amino]-1,2-diphenylethan**

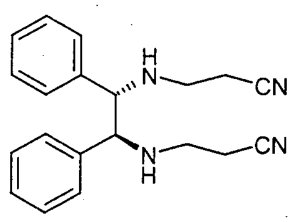

0,5 g (1S,2S)-(-)-Diphenylethylendiamin (2,4 mmol) werden in 10 ml Ethanol gelöst. Nach Zugabe von 0,32 ml Acrylnitril (4,9 mmol) wird 20 h bei Raumtemperatur gerührt. Nach dieser Zeit werden noch weitere 0,1 ml Acrylnitril (0,08 g, 1,5 mmol) zugegeben und weitere 72 h bei Raumtemperatur gerührt. Das Lösungsmittel wird abdestilliert und das verbleibende Öl mit 5 ml Ether versetzt. Der ausgefallene Feststoff (0,46 g) wird abgesaugt und im Hochvakuum getrocknet. Das Produkt besitzt aufgrund einer gaschromatographischen Analyse eine Reinheit von 99 %.

**Beispiel 3:**

**Herstellung von (1S,2S)-Bis-[N-(2-Carboxylethyl-ethyl)-amino]-cyclohexan**

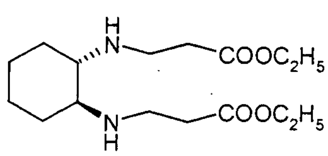

Es werden 1,0 g (1S,2S)-(-)-Diaminocyclohexan (8,8 mmol) in 50 ml Ethanol gelöst, danach werden 2,0 ml Acrylsäureethylester (1,84 g, 18,4 mmol) zugegeben. Der Ansatz wird 24 h bei Raumtemperatur gerührt. Laut gaschromatographischer Analyse ist das Edukt vollständig umgesetzt worden. Nach Abdestillieren des Lösungsmittels erhält man 2,8 g eines farblosen Öls, welches nach gaschromatographischer Analyse eine Reinheit von 96 % besitzt.

**Beispiel 4:**

**Herstellung von (1S,2S)-Bis-[N-(2-diethylphosphonato-ethyl)-amino]-cyclohexan**

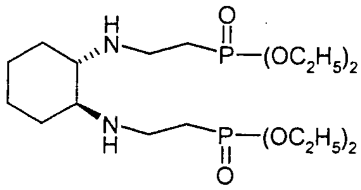

Es werden 1,0 g (1S,2S)-(-)-Diaminocyclohexan (8,8 mmol) in 10 ml Ethanol gelöst, danach werden 2,7 ml Vinylphosphonsäurediethylester (2,9 g, 17,5 mmol) zugegeben. Der Ansatz wird 27 h bei Rückflusstemperatur gerührt. Laut gaschromatographischer Analyse ist das Edukt vollständig umgesetzt worden. Nach Abdestillieren des Lösungsmittels erhält man 3,9 g eines farblosen Öls, welches nach gaschromatographischer Analyse eine Reinheit von 90 % besitzt.

**Beispiel 5:**

**Herstellung von (1S,2S)-Bis-[N-(2-diethylphosphonato-ethyl)-amino]-1,2-diphenylethan**

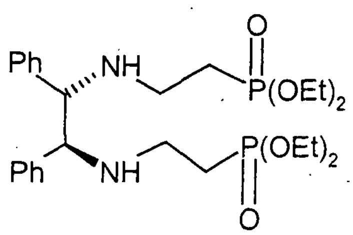

2,0 g (1S,2S)-Diphenylethylendiamin (9,4 mmol) werden in 50 ml Ethanol vorgelegt, danach werden 3,2 ml Vinylphosphonsäurediethylester (20,7 mmol) zugegeben und die Mischung 72 h auf Rückflusstemperatur erhitzt. Das Ethanol wird abdestilliert und der Rückstand über Kieselgel chromatographiert. Elution mit Essigsäureethylester/Methanol (5:2) ergibt 2,5 g Rohprodukt, welches noch Vinyl-phosphonsäureester enthält. Dieses Material wird bei 60 bis 90°C und 0,1 mbar im Kugelrohr abdestilliert. Der Rückstand enthält sauberen Liganden ($^1$H-NMR).

**Beispiel 6:**

**Herstellung von (1S,2S)-Bis-[N-(2-Carboxyethyl-ethyl)-amino]-1,2-diphenylethan**

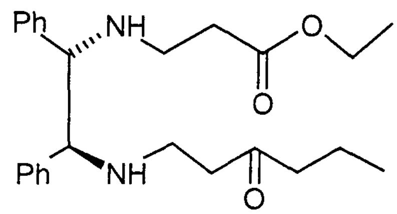

2,0 g (1S,2S)-Diphenylethylendiamin (9,4 mmol) werden in 50 ml Ethanol gelöst. Nach Zugabe von 2,2 ml Acrylsäureethylester (20,7 mmol) wird die Mischung 48 h bei Raumtemperatur gerührt. Ethanol und überschüssiger Acrylester werden im Vakuum abdestilliert. Als Rückstand verbleibt der gewünschte Ligand als einheitliche Verbindung.

**Beispiele 7 bis 10**

**Asymmetrische Reduktion von Propiophenon**

Ein Rollrandgefäß mit Septumverschluss wird evakuiert und mit Argon gefüllt. Dann werden 0,23 mmol eines chiralen Liganden der Beispiele 1 bis-6 eingewogen, in 0,7 ml Toluol gelöst und 0,21 ml einer 1,1M Lösung von Diethylzink in Toluol (0,23 mmol) zugegeben. Man lässt 10 min bei Raumtemperatur rühren, um den Komplex aus der Zinkverbindung und dem Liganden zu bilden. Danach gibt man 1,5 ml Propiophenon (1,51 g, 11,3 mmol) und 0,90 g Polymethylhydrosiloxan (PMHS, 13,2 mmol) zum Reaktionsgemisch und lässt 23 h bei 30°C rühren.
Für die analytische Untersuchung werden 0,05 ml des Reaktionsgemisches vorsichtig auf 1,5 ml einer 45 %igen wässrigen KOH getropft. Man gibt 4 ml Toluol hinzu und extrahiert das Reaktionsprodukt in die organische Phase. Nach Abtrennen der organischen Phase und Trocknen über $MgSO_4$ wird eine gaschromatographische Analyse auf einer chiralen Säule vom Typ Chirasil® durchgeführt.
Die Ergebnisse sind in Tabelle 1 dargestellt.

| Beispiel | Ligand | Umsatz (%) | % ee |
|---|---|---|---|
| 7 | 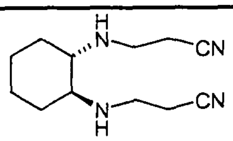 | 68 | 82 |
| 8 | 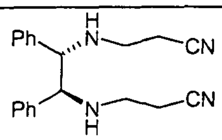 | 58 | 82 |
| 9 | 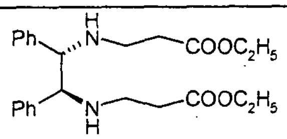 | 78 | 83 |
| 10 (nicht erfindungsgemäß) | 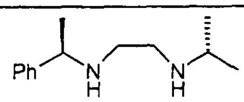 | 63 | 80 |

**Beispiele 11 bis 14**

**Asymmetrische Reduktion von Isobutyrophenon**

Ein Rollrandgefäß mit Septumverschluss wird evakuiert und mit Argon gefüllt. Dann werden 0,20 mmol eines chiralen Liganden der Beispiele 1 bis 6 eingewogen, in 0,62 ml Toluol gelöst und 0,185 ml einer 1,1M Lösung von Diethylzink in Toluol (0,20 mmol) zugegeben. Man lässt 10 min bei Raumtemperatur rühren, um den Komplex aus der Zinkverbindung und dem Liganden zu bilden. Danach gibt man 1,5 ml Isobutyrophenon (1,48 g, 10.0 mmol) und 0,80 g Polymethylhydrosiloxan (PMHS, 12,3 mmol) zum Reaktionsgemisch und lässt 23,5 h bei 30°C rühren.

Für die analytische Untersuchung werden 0,05 ml des Reaktionsgemisches vorsichtig auf 1,5 ml einer 45 %igen wässrigen KOH getropft. Man gibt 4 ml Toluol hinzu und extrahiert das Reaktionsprodukt in die organische Phase. Nach Abtrennen der organischen Phase und Trocknen über $MgSO_4$ wird eine gaschromatographische Analyse auf einer chiralen Säule vom Typ Chirasil® durchgeführt.

Die Ergebnisse sind in Tabelle 2 dargestellt.

| Beispiel | Ligand | Umsatz (%) | % ee |
|---|---|---|---|
| 11 | 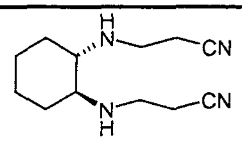 | 64 | 83 |
| 12 | 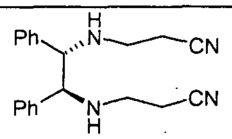 | 50 | 86 |
| 13 | 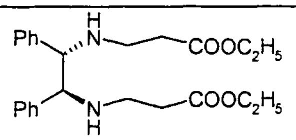 | 63 | 89 |
| 14 (nicht erfingungsgemäß) | 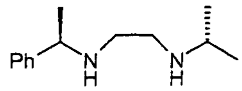 | 61 | 81 |

**Beispiele 15 bis17**

**Asymmetrische Reduktion von 2-Bromacetophenon**

Ein Rollrandgefäß mit Septumverschluss wird evakuiert und mit Argon gefüllt. Dann werden 0,20 mmol eines chiralen Liganden der Beispiele 1 bis 6 eingewogen, in 0,7 ml Toluol gelöst und 0,21 ml einer 1,1M Lösung von Diethylzink in Toluol (0,20 mmol) zugegeben. Man lässt 10 min bei Raumtemperatur rühren, um den Komplex aus der Zinkverbindung und dem Liganden zu bilden. Danach gibt man 1,52 ml 2-Bromacetophenon (2,2 g, 11,3 mmol) und 0,90 g Polymethylhydrosiloxan (PMHS, 13.2 mmol) zum Reaktionsgemisch und lässt 23 h bei 30°C rühren.

Für die analytische Untersuchung werden 0,05 ml des Reaktionsgemisches vorsichtig auf 1,5 ml einer 45 %igen wässrigen KOH getropft. Man gibt 4 ml Toluol hinzu und extrahiert das Reaktionsprodukt in die organische Phase. Nach Abtrennen der organischen Phase und Trocknen über $MgSO_4$ wird eine gaschromatographische Analyse auf einer chiralen Säule vom Typ Chirasil® durchgeführt.

Die Ergebnisse sind in Tabelle 3 dargestellt.

| Beispiel | Ligand | Umsatz (%) | % ee |
|---|---|---|---|
| 15 | 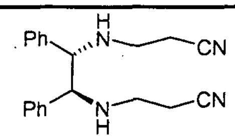 | 65 | 76 |
| 16 | 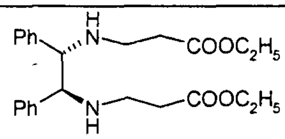 | 76 | 75 |
| 17 (nicht erfindungsgemäß) | 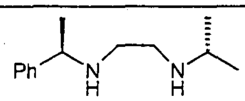 | 73 | 71 |

**Beispiele 18 bis 22**

**Asymmetrische Reduktion von 2-Methylacetophenon**

Ein Rollrandgefäß mit Septumverschluss wird evakuiert und mit Argon gefüllt. Dann werden 0,20 mmol eines chiralen Liganden der Beispiele 1 bis 6 eingewogen, in 0,62 ml Toluol gelöst und 0,185 ml einer 1,1M Lösung von Diethylzink in Toluol (0,20 mmol) zugegeben. Man lässt 10 min bei Raumtemperatur rühren, um den Komplex aus der Zinkverbindung und dem Liganden zu bilden. Danach gibt man 1,31 ml 2-Methylacetophenon (1,34 g, 10,0 mmol) und 0,80 g Polymethylhydrosiloxan (PMHS, 11,8 mmol) zum Reaktionsgemisch und lässt 22 h bei 30°C rühren.

Für die analytische Untersuchung werden 0,05 ml des Reaktionsgemisches vorsichtig auf 1,5 ml einer 45 %igen wässrigen KOH getropft. Man gibt 4 ml Toluol hinzu und extrahiert das Reaktionsprodukt in die organische Phase. Nach Abtrennen der organischen Phase und Trocknen über $MgSO_4$ wird eine gaschromatographische Analyse auf einer chiralen Säule vom Typ Chirasil® durchgeführt.

Die Ergebnisse sind in Tabelle 4 dargestellt.

| Beispiel | Ligand | Umsatz (%) | % ee |
|---|---|---|---|
| 18 | 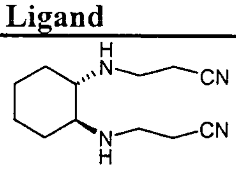 | 85 | 57 |
| 19 | 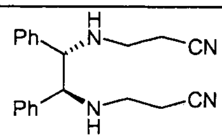 | 72 | 60 |
| 20 | 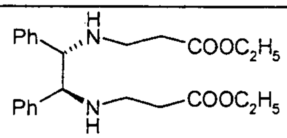 | 72 | 72 |
| 21 | 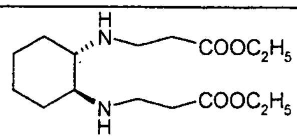 | 82 | 61 |
| 22 (nicht erfindungsgemäß) | 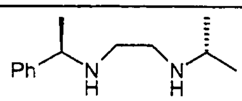 | 83 | 28 |

**Patentansprüche**

**1.** Verbindungen der Formel (I),

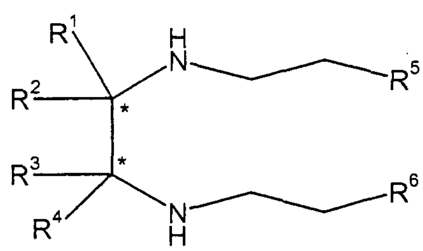

(I),

in der

| | |
|---|---|
| * | stereogene Kohlenstoffatome markiert, die jeweils unabhängig voneinander R- oder S-konfiguriert sind, wobei jedoch Meso-Formen ausgeschlossen sind und |
| $R^1$, $R^2$, $R^3$ und $R^4$ | jeweils unabhängig voneinander für Wasserstoff, $C_1$-$C_{12}$-Alkyl, $C_4$-$C_{24}$-Aryl oder $C_5$-$C_{25}$-Arylalkyl stehen oder $R^1$, $R^2$, $R^3$ und $R^4$ zusammen mit der Ethylenbrücke für 1,2-($C_5$-$C_8$-Cycloalkyl) stehen und |
| $R^5$ und $R^6$ | jeweils unabhängig voneinander für Reste stehen, die ausgewählt sind aus der Gruppe -$COOR^7$, -$CONR^8R^9$, -CN oder -$PO(OR^{10})_2$, wobei $R^7$, $R^8$, $R^9$ und $R^{10}$ jeweils für $C_1$-$C_{12}$-Alkyl, $C_4$-$C_{24}$-Aryl oder $C_5$-$C_{25}$-Arylalkyl stehen oder $NR^8R^9$ als Ganzes für einen cyclischen Aminorest mit insgesamt 4 bis 12 Kohlenstoffatomen steht. |

**2.** Verbindungen gemäß Anspruch 1, **dadurch gekennzeichnet, dass** $R^1$, $R^2$, $R^3$ und $R^4$ jeweils unabhängig voneinander für Wasserstoff, $C_1$-$C_8$-Alkyl oder $C_4$-$C_{24}$-Aryl stehen oder $R^1$, $R^2$, $R^3$ und $R^4$ zusammen mit der Ethylenbrücke für 1,2-Cyclohexylen stehen.

**3.** Verbindungen gemäß mindestens einem der Ansprüche 1 und 2, **dadurch gekennzeichnet, dass** $R^1$, $R^2$, $R^3$ und $R^4$ zusammen mit der Ethylenbrücke für (R,R)- und (S,S)-1,2-Diphenyl-1,2-ethylen oder (R,R)- und (S,S)-1,2-Cyclohexylen stehen.

**4.** Verbindungen gemäß mindestens einem der Ansprüche 1 bis 3, **dadurch gekennzeichnet, dass** $R^5$ und $R^6$ jeweils unabhängig voneinander ausgewählt sind aus der Gruppe -$COOR^7$, -$CONR^8R^9$, -CN oder -$PO(OR^{10})_2$, wobei $R^7$, $R^8$, $R^9$ und $R^{10}$ jeweils für $C_1$-$C_4$-Alkyl oder $C_4$-$C_{24}$-Aryl stehen.

**5.** (1S,2S)- und (1R,2R)-Bis-[N-(2-dimethylphosphonato-ethyl)amino]-cyclohexan, (1S,2S)- und (1R,2R)-Bis-[N-(2-diethylphosphonato-ethyl)amino]-cyclohexan, (1S,2S)- und (1R,2R)-Bis-[N-(2-diphenylphosphonato-ethyl)-amino]-cyclohexan, (1S,2S)- und (1R,2R)-Bis-[N-(2-cyanoethyl)amino]-cyclohexan, (1S,2S)- und (1R,2R)-Bis-[N-(2-carboxylethyl-ethyl)amino]-cyclohexan und (1S,2S)- und (1R,2R)-Bis-[N-(2-carboxylmethyl-ethyl)-amino]-cyclohexan, (1S,2S)- und (1R,2R)-Bis-[N-(2-dimethylphosphonatoethyl)amino]-1,2-diphenylethan, (1S,2S)- und (1R,2R)-Bis-[N-(2-diethylphosphonato-ethyl)amino]-1,2-diphenylethan, (1S,2S)- und (1R,2R)-Bis-[N-(2-diphenylphosphonato-ethyl)amino]-1,2-diphenylethan, (1S,2S)- und (1R,2R)-Bis-[N-(2-cyanoethyl)amino]-1,2-diphenylethan, (1S,2S)- und (1R,2R)-Bis-[N-(2-carboxyethyl-ethyl)amino]-1,2-diphenylethan, (1S,2S)-und (1R,2R)-Bis-[N-(2-carboxymethyl-ethyl)amino]-1,2-diphenylethan.

**6.** Übergangsmetallkomplexe von Verbindungen gemäß mindestens einem der Ansprüche 1 bis 5.

**7.** Übergangsmetallkomplexe gemäß Anspruch 6, **dadurch gekennzeichnet, dass** das Verhältnis von Übergangsmetall zu Verbindungen der Formel (I) 0,5 bis 1,5 beträgt.

**8.** Übergangsmetallkomplexe gemäß mindestens einem der Ansprüche 6 und 7, **dadurch gekennzeichnet, dass** die Verbindungen Zink- und Cobalt-Komplexe sind.

**9.** Übergangsmetallkomplexe gemäß mindestens einem der Ansprüche 6 bis 8, **dadurch gekennzeichnet, dass** die Übergangsmetallkomplexe durch Umsetzung von Zink- oder Kobalt-Halogeniden, Carbonaten, Cyanuraten, Isocyanaten, Sulfaten, Phosphaten, Nitraten, Carboxylaten oder Alkoxiden mit Verbindungen gemäß mindestens einem der Ansprüche 1 bis 5 erhältlich sind.

**10.** Übergangsmetallkomplexe gemäß Anspruch 9, **dadurch gekennzeichnet, dass** weiterhin die Umsetzung mit einem Reduktionsmittel erfolgt.

**11.** Übergangsmetallkomplexe gemäß mindestens einem der Ansprüche 6 bis 8, **dadurch gekennzeichnet, dass** die Übergangsmetallkomplexe durch Umsetzung von Zinkverbindungen $ZnY_2$ oder ZnYHal, wobei Y jeweils unabhängig für Wasserstoff, $BH_4$ oder einen organischen Rest und Hal für Brom, Chlor oder Iod stehen, mit Verbindungen gemäß mindestens einem der Ansprüche 1 bis 5 hergestellt werden.

**12.** Katalysatoren ,enthaltend Übergangsmetallkomplexe gemäß mindestens einem der Ansprüche 6 bis 11.

**13.** Verfahren zur asymmetrischen Reduktion von Ketonen mit Silanen in Gegenwart von Katalysatoren, **dadurch gekennzeichnet, dass** als Katalysatoren solche gemäß Anspruch 12 eingesetzt werden.

**14.** Verfahren gemäß Anspruch 13, **dadurch gekennzeichnet, dass** als Silane solche der Formel (V)

$$H_rSiCl_s(C_1\text{-}C_8\text{-Alkyl})_t(C_1\text{-}C_8\text{-Alkoxy})_u(\text{Phenyl})_v \quad (V),$$

in der

r eins, zwei oder drei ist

und

$$(s + t + u + v) = (4 - r)$$

ist
oder Polymethylhydrosiloxan (PMHS) mit der wiederkehrenden Struktureinheit

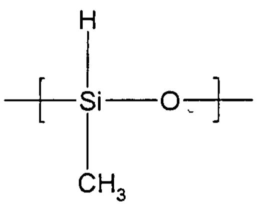

,

verwendet werden.

**15.** Verfahren gemäß mindestens einem der Ansprüche 13 und 14, **dadurch gekennzeichnet, dass** die Menge des Katalysators so gewählt wird, dass das molare Verhältnis von Übergangsmetall zu eingesetztem Keton 0,01 bis 0,20 beträgt.

**16.** Verfahren gemäß mindestens einem der Ansprüche 13 bis 15, **dadurch gekennzeichnet, dass** als Ketone Arylketone eingesetzt werden.

**17.** Verwendung von stereoisomerenangereicherten Alkoholen, die nach einem Verfahren gemäß mindestens einem der Ansprüche 13 bis 16 hergestellt wurden, in einem Verfahren zur Herstellung von Arzneimitteln und Agrochemikalien.

Europäisches Patentamt

# EUROPÄISCHER RECHERCHENBERICHT

Nummer der Anmeldung
EP 03 01 8220

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,X | MIMOUN H ET AL: "ENANTIOSELECTIVE REDUCTION OF KETONES BY POLYMETHYLHYDROSILOXANE INTHE PRESENCE OF CHIRAL ZINC CATALYSTS" JOURNAL OF THE AMERICAN CHEMICAL SOCIETY, AMERICAN CHEMICAL SOCIETY, WASHINGTON, DC, US, Bd. 121, Nr. 26, 1999, Seiten 6158-6166, XP001034278 ISSN: 0002-7863 * Seite 6158, Spalte 1, Zeile 1 - Zeile 4 * | 17 | C07C29/143 C07C67/56 C07C237/06 C07C255/25 C07F9/40 C07F15/00 |
| A | | 1-16 | |
| | ----- | | |
| D,A | WO 00 24751 A (FAIVRE CHAUVET ALAIN ; GESTIN JEAN FRANCOIS (FR); I N S E R M INST NAT) 4. Mai 2000 (2000-05-04) * Seite 18; Beispiele 2a,2b,2d * | 1-16 | |
| | ----- | | |
| A | SAKATA K ET AL.: "Preparation and Spectral Properties of Nickel(II) and Copper(II) Complexes with Two Isomeric Cyclohexylcyclams" POLYHEDRON, Bd. 17, Nr. 9, 1998, Seiten 1463-1470, XP0001156620 * Beispiel 1t * | 1-16 | RECHERCHIERTE SACHGEBIETE (Int.Cl.7) C07C C07F |
| | ----- | | |
| A | DE 23 38 216 A (EXXON RESEARCH ENGINEERING CO) 14. Februar 1974 (1974-02-14) * Seite 8, letzte Verbindung * | 1-16 | |
| | ----- | | |

Der vorliegende Recherchenbericht wurde für alle Patentansprüche erstellt

| Recherchenort | Abschlußdatum der Recherche | Prüfer |
|---|---|---|
| München | 2. Dezember 2003 | Janus, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 03 01 8220

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-12-2003

| Im Recherchenbericht angeführtes Patentdokument | Datum der Veröffentlichung | Mitglied(er) der Patentfamilie | Datum der Veröffentlichung |
|---|---|---|---|
| WO 0024751 A | 04-05-2000 | AU 1043000 A | 15-05-2000 |
| | | CA 2346976 A1 | 04-05-2000 |
| | | WO 0024751 A1 | 04-05-2000 |
| | | EP 1123301 A1 | 16-08-2001 |
| | | JP 2002528458 T | 03-09-2002 |
| DE 2338216 A | 14-02-1974 | CH 602527 A5 | 31-07-1978 |
| | | DE 2338216 A1 | 14-02-1974 |
| | | JP 49055629 A | 30-05-1974 |
| | | US 4088666 A | 09-05-1978 |
| | | US 4156603 A | 29-05-1979 |
| | | US 4165330 A | 21-08-1979 |

EPO FORM P0461

Für nähere Einzelheiten zu diesem Anhang : siehe Amtsblatt des Europäischen Patentamts, Nr.12/82